# FASCICULE DE BREVET EUROPEEN

(11) **EP 0 827 738 B1**
(45) Date de publication et mention de la délivrance du brevet: **03.11.1999**
(21) Numéro de dépôt: 97401997.8
(22) Date de dépôt: 27.08.1997
(51) Int. Cl.: A61K 7/06, A61K 7/13

(54) **Composition de teinture d'oxydation pour fibres kératiniques comprenant un polymère amphiphile anionique**
Zusammensetzung zum oxidativen Färben von Keratinfasern, die ein anionisches, amphiphiles Polymer enthält
Composition for the oxidative dyeing of keratinic fibers containing an amphiphilic anionic polymer

(30) Priorité: 06.09.1996 FR 9610921
(43) Date de publication de la demande: 11.03.1998
(73) Titulaire: L'OREAL, 75008 Paris (FR)
(72) Inventeur: De La Mettrie, Roland, 78110 Le Vesinet (FR); Boudy, Françoise, 75012 Paris (FR)
(74) Mandataire: Miszputen, Laurent

(56) Documents cités:
- WO-A-91/15186
- WO-A-91/15187
- FR-A- 2 327 761
- FR-A- 2 679 444

## Description

La présente invention concerne une composition de teinture d'oxydation des fibres kératiniques et en particulier des fibres kératiniques humaines telles que les cheveux, comprenant au moins un précurseur de colorant d'oxydation et éventuellement un ou plusieurs coupleurs et au moins un polymère amphibile anionique comportant au moins un motif hydrophile de formule (I) ci-aprés définie, et au moins un motif hydrophobe exclusivement ester d'alkyl (C₁₀-C₃₀) d'acide carboxylique insaturé.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des 〈〈bases d'oxydation〉〉 sur elles-mêmes, soit d'une condensation oxydative des 〈〈bases d'oxydation〉〉 sur des composés modificateurs de coloration, ou 〈〈coupleurs〉〉, qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les 〈〈bases d'oxydation〉〉 et d'autre part par les 〈〈coupleurs〉〉, permet l'obtention d'une palette très riche en coloris.

Pour localiser le produit de coloration d'oxydation à l'application sur les cheveux afin qu'il ne coule pas sur le visage ou en dehors des zones que l'on se propose de tendre, on a jusqu'ici eu recours à l'emploi d'épaississants traditionnels tels que l'acide polyacrylique réticulé, les hydroxyéthylcelluloses, les cires ou encore à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.

Cependant, la demanderesse a constaté que les ingrédients du type épaississants traditionnels, tensio-actifs et solvants, freinent généralement la montée du colorant sur les fibres, ce qui se traduit par une nuance terne et aussi par une utilisation plus importante de colorant, de solvant et/ou d'agents tensioactifs pour solubiliser le colorant, si l'on veut néanmoins obtenir une nuance puissante.
Par ailleurs, elle a également constaté qu'après mélange avec l'oxydant, les compositions tinctoriales contenant le ou les précurseurs de colorants d'oxydation et éventuellement le ou les coupleurs, et en outre lesdits ingrédients, perdaient une partie de leur caractère gélifié et engendraient par voie de conséquence des coulées indésirables.

La demande de brevet EP-769 290 décrit l'utilisation de polymères amphiphiles ioniques comportant au moins une chaîne grasse et au moins un motif hydrophile en association avec au moins un polymère siliconé, dans une composition cosmétique que l'on applique sur les cheveux dans le but d'en améliorer les propriétés cosmétiques, et notamment la douceur au toucher, puis que l'on rince à l'eau; ledit traitement cosmétique des cheveux peut s'effectuer, avant de colorer les cheveux, ou après les avoir colorés; c'est-à-dire que, dans le cas d'une coloration d'oxydation, on applique sur les cheveux, la composition selon EP-769 290 puis on rince, ce traitement ayant lieu, (a), avant ou après avoir appliqué le mélange extemporané [colorant (1) + oxydant (2)] sur les cheveux, et (b) dans le cas d'une coloration d'oxydation en deux temps [application du colorant (1) suivie de l'application de l'oxydant (2)], avant d'appliquer le colorant (1) ou après avoir appliqué l'oxydant (2) sur les cheveux.

Or, après d'importantes recherches menées sur la question, la Demanderesse vient maintenant de découvrir qu'il est possible d'obtenir des compositions de teinture d'oxydation (après mélange avec l'oxydant) qui ne coulent pas et restent donc mieux localisées au point d'application, et qui permettent aussi d'obtenir des nuances plus chromatiques (plus lumineuses) et plus puissantes, si on introduit (i) soit dans la composition contenant le ou les précurseurs de colorants d'oxydation et éventuellement le ou les coupleurs [ou composition (A)], soit (ii) dans la composition oxydante [ou composition (B)], ou (iii) dans les deux compositions à la fois, une quantité efficace d'un polymère amphiphile anionique comportant au moins un motif hydrophile de formule (I) : 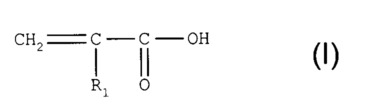 dans laquelle R₁ désigne H ou CH₃ ou C₂H₅, c'est-à-dire des motifs acide acrylique, acide méthacrylique ou acide éthacrylique,
et au moins un motif hydrophobe exclusivement ester d'alkyl (C₁₀-C₃₀) d'acide carboxylique insaturé.

Au sens de la présente invention, la chromaticité (luminosité) est définie par la valeur **c*** dans le système de notation colorimétrique L*, a*, b*, de la Commission Internationale de l'Eclairage (C.I.E.). Cette valeur est égale à la racine carrée de la somme a² + b² (**+**a est rouge, -a est vert, **+**b est jaune, -b est bleu). La nuance est d'autant plus lumineuse que la valeur de c* est grande. Dans ce système de notation, **L*** définit la puissance de la nuance. La nuance est d'autant plus puissante que la valeur de L* est faible (0=noir, 100=blanc).

Ces découvertes sont à la base de la présente invention.

La présente invention a ainsi pour objet une composition de teinture d'oxydation pour fibres kératiniques, en particulier pour fibres kératiniques humaines, telles que les cheveux, du type comprenant, dans un milieu approprié pour la teinture, au moins un précurseur de colorant d'oxydation (base d'oxydation) et, le cas échéant, un ou plusieurs coupleurs, qui est caractérisée par le fait qu'elle contient en outre au moins un polymère amphiphile anionique comportant au moins un motif hydrophile de formule (I) ci-avant définie, et au moins un motif hydrophobe exclusivement ester d'alkyl (C₁₀-C₃₀) d'acide carboxylique insaturé.

Gràce à la présente invention, il est en outre possible, et ceci de manière avantageuse, de réduire la consommation en agents tensio-actifs, voire de les supprimer.
L'invention permet également de diminuer la quantité de matières actives colorantes utilisées dans les compositions de teinture, par rapport aux techniques classiques et connues de l'art antérieur.

Un autre objet de la présente invention porte sur une composition prête à l'emploi pour la teinture des fibres kératiniques, qui contient au moins un précurseur de colorant d'oxydation et éventuellement au moins un coupleur, et au moins un polymère amphiphile anionique comportant au moins un motif hydrophile de formule (I) ci-avant définie, et au moins un motif hydrophobe exclusivement ester d'alkyl (C₁₀-C₃₀) d'acide carboxylique insaturé, et un agent oxydant.

L'invention vise également un procédé de teinture des fibres kératiniques, et en particulier des fibres kératiniques humaines telles que les cheveux, consistant à appliquer sur ces fibres au moins une composition (A1) contenant, dans un milieu approprié pour la teinture, au moins un précurseur de colorant d'oxydation et éventuellement au moins un coupleur, en association avec au moins un polymère amphiphile anionique comportant au moins un motif hydrophile de formule (I) ci-avant définie, et au moins un motif hydrophobe exclusivement ester d'alkyl (C₁₀-C₃₀) d'acide carboxylique insaturé, la couleur étant révélée à pH alcalin, neutre ou acide à l'aide d'un agent oxydant qui est mélangé juste au moment de l'emploi à la composition (A1) ou qui est présent dans une composition (B1) appliquée séquentiellement sans rinçage intermédiaire.
L'invention vise aussi une variante de ce procédé, qui consiste à appliquer sur les fibres au moins une composition (A2) contenant dans un milieu approprié pour la teinture, au moins un précurseur de colorant d'oxydation et éventuellement au moins un coupleur, et ceci en la présence ou l'absence de polymère amphiphile anionique comportant au moins un motif hydrophile de formule (I) ci-avant définie, et au moins un motif hydrophobe exclusivement ester d'alkyl (C₁₀-C₃₀) d'acide carboxylique insaturé, la couleur étant révélée à pH alcalin, neutre ou acide à l'aide d'une composition oxydante (B2) qui contient un agent oxydant et une quantité effficace d'au moins un polymère amphiphile anionique comportant au moins un motif hydrophile de formule (I) ci-avant, et au moins un motif hydrophobe exclusivement ester d'alkyl (C₁₀-C₃₀) d'acide carboxylique insaturé, et qui est mélangée juste au moment de l'emploi à la composition (A2) ou qui est appliquée séquentiellement sans rinçage intermédiaire.

L'invention a également pour objet des dispositifs de teinture ou 〈〈 kits 〉〉 à plusieurs compartiments, dont le premier compartiment contient au moins un précurseur de colorant d'oxydation, éventuellement au moins un coupleur, et au moins un polymère amphiphile anionique comportant au moins un motif hydrophile de formule (I) ci-avant définie, et au moins un motif hydrophobe exclusivement de type ester d'alkyl (C₁₀-C₃₀) d'acide carboxylique insaturé, et le deuxième compartiment un agent oxydant.
Selon une autre variante, l'invention a également pour objet des dispositifs de teinture ou 〈〈 kits 〉〉 à plusieurs compartiments, dont le premier compartiment contient au moins un précurseur de colorant d'oxydation, éventuellement au moins un coupleur, et ceci en la présence ou en l'absence de polymère amphiphile anionique comportant au moins un motif hydrophile de formule (I) ci-avant définie, et au moins un motif hydrophobe exclusivement ester d'alkyl (C₁₀-C₃₀) d'acide carboxylique insaturé, et le deuxième compartiment un agent oxydant et une quantité efficace d'au moins un polymère amphiphile anionique comportant au moins un motif hydrophile de formule (I) ci-avant définie, et au moins un motif hydrophobe exclusivement ester d'alkyl (C₁₀-C₃₀) d'acide carboxylique insaturé.

L'invention concerne aussi l'utilisation de la composition de teinture d'oxydation ci-dessus définie ou d'un dispositif de teinture ou 〈〈 kit 〉〉 à plusieurs compartiments tel que défini ci-avant pour la teinture des fibres kératiniques humaines telles que les cheveux.

Mais d'autres caractéristiques, aspects, objets et avantages de l'invention apparaîtront encore plus clairement à la lecture de la description et des exemples qui suivent.

Les polymères amphiphiles anioniques comportant au moins un motif hydrophile de formule (I) ci-avant définie, et au moins un motif hydrophobe exclusivement ester d'alkyl (C₁₀-C₃₀) d'acide carboxylique insaturé, utilisés selon l'invention, sont choisis de préférence parmi ceux dont le motif hydrophobe ester d'alkyl (C₁₀-C₃₀) d'acide carboxylique insaturé correspond au monomère de formule (II) suivante : 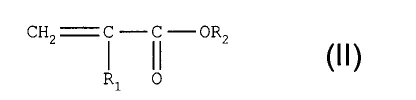 formule dans laquelle, R₁ désigne H ou CH₃ ou C₂H₅ (c'est-à-dire des motifs acrylates, méthacrylates ou éthacrylates) et de préférence H (motifs acrylates) ou CH₃ (motifs méthacrylates), R₂ désignant un radical alkyle en C₁₀-C₃₀, et de préférence en C₁₂-C₂₂.

Des esters d'alkyls (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères amphiphiles anioniques de ce type sont par exemple décrits et préparés, selon les brevets US-3 915 921 et 4 509 949.

Les polymères amphiphiles anioniques utilisables dans le cadre de la présente invention peuvent désigner plus particulièrement des polymères formés à partir d'un mélange de monomères comprenant :
(i) essentiellement de l'acide acrylique, un ester de formule (II) suivante : 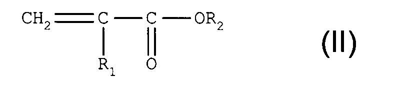 dans laquelle R₁ désigne H ou CH₃, R₂ désignant un radical alkyle ayant de 12 à 22 atomes de carbone, et un agent réticulant, tels que par exemple ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant,
(ii) essentiellement de l'acide acrylique et du méthacrylate de lauryle tel que celui formé à partir de 66% en poids d'acide acrylique et 34% en poids de méthacrylate de lauryle.

Ledit réticulant est un monomère contenant un groupe 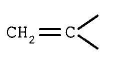 avec au moins un autre groupement polymérisable dont les liaisons insaturées sont non conjuguées l'une par rapport à l'autre. On peut notamment citer les polyallyléthers tels que notamment le polyallylsucrose et le polyallylpentaérythritol.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.C. sous la dénomination COATEX SX.

Les polymères amphiphiles anioniques comportant au moins un motif hydrophile de formule (I) ci-avant définie, et au moins un motif hydrophobe exclusivement ester d'alkyl (C₁₀-C₃₀) d'acide carboxylique insaturé, sont utilisés selon l'invention de préférence en une quantité pouvant varier d'environ 0,05 à 10% en poids du poids total de la composition de teinture appliquée sur les fibres. Plus préférentiellement, cette quantité varie d'environ 0,2 à 5% en poids.

Les précurseurs de colorants d'oxydation utilisables dans le cadre de la présente invention sont choisis parmi ceux classiquement connus en teinture d'oxydation, et parmi lesquels on peut notamment citer :
- les paraphénylènediamines de formule (III) suivante et leurs sels d'addition avec un acide : 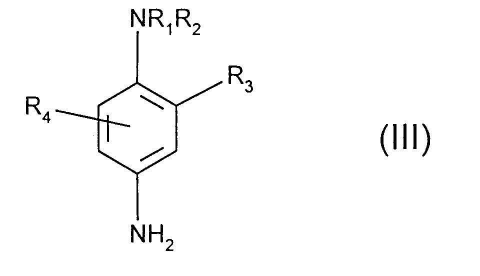 dans laquelle :
   **R**_{**1**} représente un atome d'hydrogène, un radical alkyle en C₁-C₄, monohydroxyalkyle en C₁-C₄, polyhydroxyalkyle en C₂-C₄ ou 4'-aminophényle,
   **R**_{**2**} représente un atome d'hydrogène, un radical alkyle en C₁-C₄, monohydroxyalkyle en C₁-C₄ ou polyhydroxyalkyle en C₂-C₄,
   **R**_{**3**} représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
   **R**_{**4**} représente un atome d'hydrogène ou un radical alkyle en C₁-C₄,

Parmi les paraphénylènediamines de formule (III) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-amino-N,N-bis-(β-hydroxyéthyl)-3-méthyl-aniline, la 4-amino-3-chloro-N,N-bis-(β-hydroxyéthyl)-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl, β-hydroxyéthyl)-paraphénylènediamine, la N-(β,γ-dihydroxypropyl)paraphénylènediamine, la N-(4'-aminophényl)-paraphénylènediamine, la N-phényl-paraphénylènediamine, la 2-β-hydroxyéthyloxy-paraphénylènediamine, et leurs sels d'addition avec un acide.

Parmi les paraphénylènediamines de formule (III) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- les bis-phénylalkylènediamines répondant à la formule (IV) suivante, et leurs sels d'addition avec un acide : 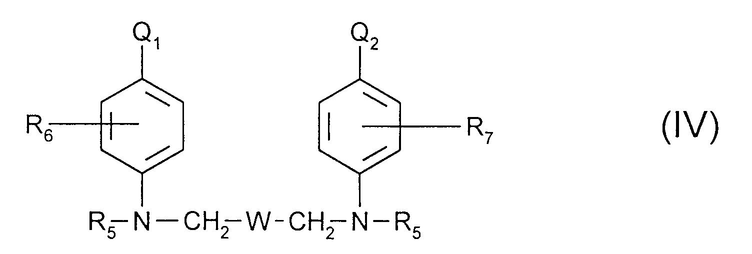 dans laquelle :
   **Q**_{**1**} et **Q**_{**2**}, identiques ou différents, représentent un radical hydroxyle ou NH**R**₈ dans lequel R₈ représente un atome d'hydrogène ou un radical alkyle en C₁-C₄,
   **R**_{**5**} représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
   **R**_{**6**} et **R**_{**7**}, identiques ou différents, représentent un atome d'hydrogène ou d'halogène ou un radical alkyle en C₁-C₄,
   **W** représente un radical pris dans le groupe constitué par les radicaux suivants : 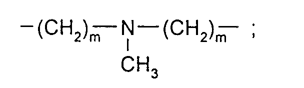 dans lesquels n est un nombre entier compris entre 0 et 8 inclusivement et m est un nombre entier compris entre 0 et 4 inclusivement.

Parmi les bis-phénylalkylènediamines de formules (IV) ci-dessus, on peut plus particulièrement citer le N,N'-bis-(β-hydroxyéthyl)-N,N'-bis-(4'-aminophényl)-1,3-diamino-2-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthylaminophényl)-tétraméthylènediamine, la N,N'-bis-(éthyl)-N,N'-bis-(4'-amino, 3'-méthylphényl)-éthylènediamine, et leurs sels d'addition avec un acide.

Parmi ces bis-phénylalkylènediamines de formule (IV), le N,N'-bis-(β-hydroxyéthyl)-N,N'-bis-(4'-aminophényl)-1,3-diamino-2-propanol ou l'un de ses sels d'addition avec un acide sont particulièrement préférés.
- les para-aminophénols répondant à la formule (V) suivante, et leurs sels d'addition avec un acide : 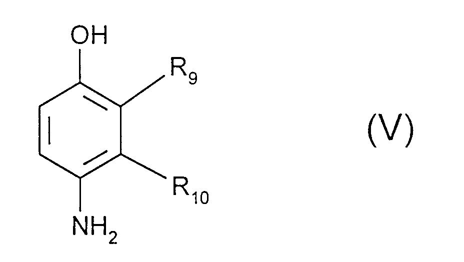 dans laquelle :
   **R**_{**9**} représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   **R**_{**10**} représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
      étant entendu qu'au moins un des radicaux R₉ ou R₁₀ représente un atome d'hydrogène.

Parmi les para-aminophénols de formule (V) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- les bases hétérocycliques utilisables à titre de bases d'oxydation dans le cadre de la présente invention, sont notamment choisies parmi les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB-1 026 978 et GB-1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE-2 359 399 ou japonais JP-88-169 571 et JP-91-333 495, comme la 2,4,5,6-tétra-aminopyrimidine, la 4-hydroxy-2,5,6-triaminopyrimidine, et leurs sels d'addition avec un acide.

Parmi les dérivés pyrazoliques, on peut plus particulièrement citer les composés décrits dans les brevets DE-3 843 892, DE-4 133 957 et demandes de brevet WO-94/08969 et WO-94/08970 comme le 4,5-diamino-1-méthyl-pyrazole, le 3,4-diamino-pyrazole, le 4,5-diamino-1-(4'-chlorobenzyl)-pyrazole, et leurs sels d'addition avec un acide.

Selon l'invention, la ou les bases d'oxydation représentent de préférence, de 0,0005 à 12% en poids environ du poids total de la composition (A) et encore plus préférentiellement de 0,005 à 6% en poids environ.

Les coupleurs utilisables dans le procédé de teinture selon l'invention sont ceux classiquement utilisés dans les compositions de teinture d'oxydation, c'est-à-dire des métaphénylènediamines, des métaaminophénols et des métadiphénols, les dérivés mono- ou poly-hydroxylés du naphtalène, le sésamol et ses dérivés et des composés hétérocycliques tels que par exemple les coupleurs indoliques, les coupleurs indoliniques, les coupleurs pyridiniques et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, et leurs sels d'addition avec un acide.

Lorsqu'ils sont présents, ces coupleurs représentent de préférence de 0,0001 à 10% en poids environ du poids total de la composition (A), et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition (A) peut encore contenir, en plus des précurseurs de colorants d'oxydation définis ci-dessus et des éventuels coupleurs associés, des colorants directs pour enrichir les nuances en reflets. Ces colorants directs peuvent notamment alors être choisis parmi les colorants nitrés, azoïques ou anthraquinoniques.

La composition (A) et/ou la composition (B) peuvent en outre plus particulièrement contenir, au moins un polymère substantif cationique ou amphotère tel que défini aux pages 3 et 4 de la demande de brevet EP-0 673 641 A1, et dont on préfère avantageusement mettre en oeuvre :
- les polymères de polyammonium quaternaire préparés et décrits dans le brevet français 2 270 846, constitués de motifs récurrents répondant à la formule (VI) suivante : 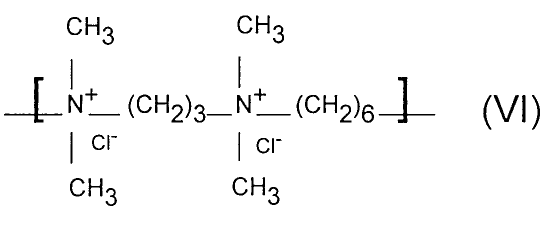 et dont le poids moléculaire, déterminé par chromatographie par perméation de gel, est compris entre 9500 et 9900;
- les polymères de polyammonium quaternaire préparés et décrits dans le brevet français 2 270 846, constitués de motifs récurrents répondant à la formule (VII) suivante : 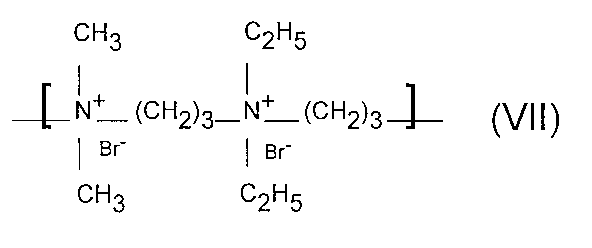 et dont le poids moléculaire, déterminé par chromatographie par perméation de gel, est d'environ 1200.

Le milieu de la composition (A) approprié pour la teinture, est de préférence un milieu aqueux constitué par de l'eau et peut éventuellement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition (A) peut encore contenir une quantité efficace d'autres agents, par ailleurs antérieurement connus en coloration d'oxydation, tels que divers adjuvants usuels comme des séquestrants, des agents de conditionnement du cheveu et en particulier des silicones, des conservateurs, des opacifiants, etc..., et éventuellement des agents tensio-actifs anioniques, non-ioniques, amphotères ou leurs mélanges.

Ladite composition peut également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter.butyl-hydroquinone et l'acide homogentisique, et ils sont alors généralement présents dans des quantités allant d'environ 0,05 à 1,5% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition (B), l'agent oxydant est choisi de préférence parmi le peroxyde d'urée, les bromates ou ferricyanures de métaux alcalins, les persels tels que les perborates, les percarbonates et les persulfates. L'utilisation du peroxyde d'hydrogène est particulièrement préférée.
La composition (B) est avantageusement constituée par une solution d'eau oxygénée dont le titre peut varier, plus particulièrement, d'environ 2,5 à 40 volumes, et encore plus préférentiellement d'environ 5 à 20.

Le pH de la composition prête à l'emploi et appliquée sur les fibres kératiniques [composition résultant du mélange de la composition tinctoriale (A) et de la composition oxydante (B)], est généralement compris entre les valeurs 4 et 11. Il est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VIII) suivante : 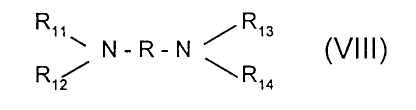 dans laquelle R est un reste propylène éventuellement substitué par un groupement hydroxyle ou un radical alkyle en C₁-C₄; R₁₁, R₁₂, R₁₃ et R₁₄, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer un mélange, réalisé extemporanément au moment de l'emploi à partir des compositions (A) et (B) décrites ci-avant, sur les fibres kératiniques sèches ou humides, et à le laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Des exemples concrets illustrant l'invention vont maintenant être donnés, sans pour autant présenter un caractère limitatif.

### EXEMPLE 1:

On a préparé la composition de teinture, conforme à l'invention, suivante :

| | |
|---|---|
| Polymère amphiphile anionique réticulé acide acrylique/acrylate d'alkyles en C₁₀-C₃₀ (PEMULEN TR1 de Goodrich) | 1,0 g |
| Acide oléïque | 3,0 g |
| Solution aqueuse de bisulfite de sodium à 35% de MA* | 0,45 g MA* |
| Paraphénylènediamine | 0,162g |
| Résorcine | 0,165g |
| Ammoniaque (20% de NH₃) | 11,5 g |
| Agent séquestrant q.s. | |
| Eau q.s.p. | 100 g |

| | |
|---|---|
| MA* = Matière Active | |

Au moment de l'emploi, on a mélangé cette composition poids pour poids avec une solution d'eau oxygénée à 20 volumes, puis on a appliqué le mélange obtenu, sur des mèches de cheveux naturels à 90% de blancs. Après 10 minutes de pause, on a rincé les mèches, puis on les a lavées avec un shampooing, rincées à nouveau, puis on les a séchées.
A l'aide d'un spectrocolorimètre I.C.S., on a mesuré la chromaticité **c***de la nuance à partir des valeurs de a* et de b* dans le système international de notation de la couleur L*, a*, b*, de la C.I.E.
- Le résultat a été le suivant :: c* = 14,36.

On a également mesuré la valeur **L***, de la nuance.
- Le résultat a été le suivant :: L* = 48,09.

### EXEMPLE 2 COMPARATIF :

On a reproduit l'exemple 1, en remplaçant 1 gramme de polymère amphiphile anionique réticulé acide acrylique/acrylate d'alkyles en C₁₀-C₃₀ (PEMULEN TR1 de Goodrich) par le mélange des deux tensio-actifs non-ioniques suivants (permettant d'obtenir la même viscosité) :
18 grammes d'alcool décylique (C₁₀-C₁₂-C₁₄ / 85-8,5-6,5) oxyéthyléné à 3,5 moles d'oxyde d'éthylène vendu sous la dénomination Mergital BL 309 par la société Henkel, et
12 grammes d'alcool décylique (C₁₀-C₁₂-C₁₄ / 85-8,5-6,5) oxyéthyléné à 5,5 moles d'oxyde d'éthylène vendu sous la dénomination Mergital BL 589 par la société Henkel.
On a ensuite suivi le même protocole qu'à l'exemple 1.
- Les résultats ont été les suivants :: c* = 12,86
L* = 49,72

### CONCLUSION :

La nuance obtenue selon l'invention est plus lumineuse ( c* plus grand) que celle obtenue selon l'art antérieur; elle est aussi plus puissante (L* plus petit).

## Revendications

1. Composition de teinture d'oxydation pour fibres kératiniques, en particulier pour fibres kératiniques humaines telles que les cheveux, comprenant dans un milieu approprié pour la teinture, au moins un précurseur de colorant d'oxydation et, le cas échéant, un ou plusieurs coupleurs, caractérisée par le fait qu'elle contient en outre au moins un polymère amphiphile anionique comportant au moins un motif hydrophile de formule (I) suivante : 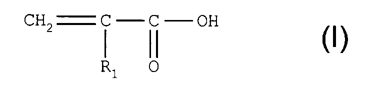 dans laquelle R₁ désigne H ou CH₃ ou C₂H₅, et au moins un motif hydrophobe exclusivement ester d'alkyl (C₁₀-C₃₀) d'acide carboxylique insaturé.

2. Composition selon la revendication 1, caractérisée par le fait que le motif hydrophile du polymère amphiphile anionique est un acide acrylique, un acide méthacrylique ou leurs mélanges.

3. Composition selon la revendication 1 ou 2, caractérisée par le fait que le motif hydrophobe du polymère amphiphile anionique est un ester d'alkyl (C₁₀-C₃₀) d'acide carboxylique insaturé de formule (II) suivante : 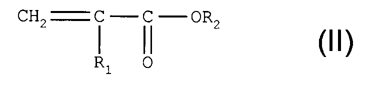 dans laquelle R₁ désigne H ou CH₃ ou C₂H₅, R₂ désignant un radical alkyle en C₁₀-C₃₀.

4. Composition selon la revendication 3, caractérisée par le fait que dans la formule (II), R₁ désigne H ou CH₃.

5. Composition selon les revendications 3 ou 4, caractérisée par le fait que dans la formule (II) R₂ désigne un radical alkyle en C₁₂-C₂₂.

6. Composition selon l'une quelconque des revendications précédentes, caractérisée par le fait que le polymère amphiphile anionique est réticulé.

7. Composition selon l'une quelconque des revendications précédentes, caractérisée par le fait que le polymère amphiphile anionique est un polymère formé à partir d'un mélange de monomères comprenant essentiellement de l'acide acrylique, un ester de formule (II) suivante : 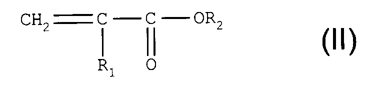 dans laquelle R₁ désigne H ou CH₃, R₂ désignant un radical alkyle ayant de 12 à 22 atomes de carbone, et, un agent réticulant.

8. Composition selon l'une quelconque des revendications 1 à 5, caractérisée par le fait que le polymère amphiphile anionique est un polymère d'acide acrylique et de méthacrylate de lauryle.

9. Composition selon l'une quelconque des revendications précédentes, caractérisée par le fait que les précurseurs de colorants d'oxydation sont choisis parmi les ortho- ou para- phénylènediamines, les bis-phénylalkylènediamines, les ortho- ou para- aminophénols, et les bases hétérocycliques, ainsi que les sels d'addition de ces composés avec un acide.

10. Composition selon l'une quelconque des revendications précédentes, caractérisée par le fait que les précurseurs de colorants d'oxydation sont présents dans des concentrations allant de 0,0005 à 12% en poids par rapport au poids total de la composition.

11. Composition selon l'une quelconque des revendications précédentes, caractérisée par le fait que les coupleurs sont choisis parmi les métaphénylènediamines, les métaaminophénols, les métadiphénols, les coupleurs hétérocycliques, et les sels d'addition de ces composés avec un acide.

12. Composition selon l'une quelconque des revendications précédentes, caractérisée par le fait que les coupleurs sont présents dans des concentrations allant de 0,0001 à 10% en poids par rapport au poids total de la composition.

13. Composition selon l'une quelconque des revendications 9 à 12, caractérisée par le fait que les sels d'addition avec un acide des précurseurs de colorants d'oxydation et des coupleurs sont choisis parmi les chlorhydrates, les bromhydrates, les sulfates, les tartrates, les lactates et les acétates.

14. Composition selon l'une quelconque des revendications précédentes, caractérisée par le fait qu'elle contient en outre des colorants directs.

15. Composition selon l'une quelconque des revendications précédentes, caractérisée par le fait qu'elle contient en outre au moins un polymère substantif cationique ou amphotère.

16. Composition selon la revendication 15, caractérisée par le fait que le polymère est un polymère de polyammonium quaternaire constitué de motifs récurrents répondant à la formule (VI) suivante :

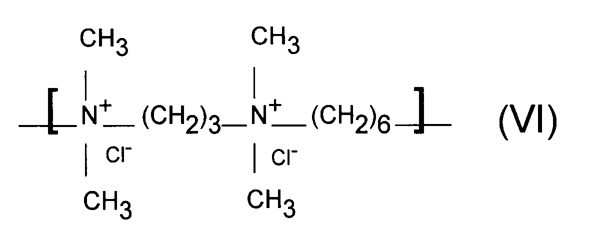

17. Composition selon la revendication 15, caractérisée par le fait que le polymère est un polymère de polyammonium quaternaire constitué de motifs récurrents répondant à la formule (VII) suivante :

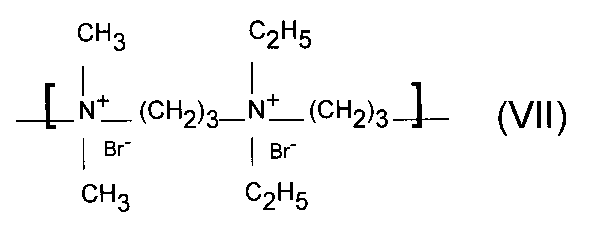

18. Composition selon l'une quelconque des revendications précédentes, caractérisée par le fait qu'elle contient en outre au moins un agent antioxydant, présent dans des quantités allant de 0,05 à 1,5% en poids par rapport au poids total de la composition.

19. Composition selon l'une quelconque des revendications précédentes, prête à l'emploi, caractérisée par le fait qu'elle contient en outre un agent oxydant.

20. Composition selon la revendication 19, caractérisée par le fait qu'elle possède un pH allant de 4 à 11.

21. Composition selon la revendication 19 ou 20, caractérisée par le fait que l'agent oxydant est choisi parmi le peroxyde d'hydrogène, le peroxyde durée, les bromates et les ferricyanures de métaux alcalins, et les persels.

22. Composition selon l'une quelconque des revendications 19 à 21, caractérisée par le fait que l'agent oxydant est une solution d'eau oxygénée dont le titre varie de 2,5 à 40 volumes.

23. Composition selon l'une quelconque des revendications précédentes, caractérisée par le fait que les polymères amphiphiles anioniques tels que définis dans l'une quelconque des revendications 1 à 8, sont utilisés en une quantité quantité allant de 0,05 à 10% en poids du poids total de la composition appliquée sur les fibres et encore plus préférentiellement de 0,2 à 5%.

24. Procédé de teinture des fibres kératiniques et en particulier des fibres kératiniques humaines telles que les cheveux, caractérisé par le fait qu'il consiste à appliquer sur les fibres une composition de teinture (A1) telle que définie à l'une quelconque des revendications 1 à 18, et à révéler la couleur en milieu alcalin neutre ou acide à l'aide d'un agent oxydant qui est ajouté juste au moment de l'emploi à cette composition (A1) ou qui est présent dans une composition (B1) appliquée séquentiellement sans rinçage intermédiaire.

25. Procédé de teinture des fibres kératiniques et en particulier des fibres kératiniques humaines telles que les cheveux, caractérisé par le fait qu'il consiste à appliquer sur les fibres une composition de teinture (A2) contenant dans un milieu approprié pour la teinture au moins un précurseur de colorant d'oxydation, éventuellement un ou plusieurs coupleurs, en la présence ou en l'absence d'un polymère amphiphile anionique tel que défini dans l'une quelconque des revendications 1 à 8, et à révéler la couleur en milieu alcalin, neutre ou acide à l'aide d'une composition oxydante (B2) contenant un agent oxydant et une quantité efficace d'au moins un polymère amphiphile anionique tel que défini dans l'une quelconque des revendications 1 à 8, et qui est mélangée juste au moment de l'emploi à la composition (A2) ou qui est appliquée séquentiellement sans rinçage intermédiaire.

26. Dispositif à plusieurs compartiments ou 〈〈 Kit 〉〉 pour la teinture des fibres kératiniques et en particulier des fibres kératiniques humaines telles que les cheveux, caractérisé par le fait qu'il comporte au moins deux compartiments, dont l'un d'entre eux renferme une composition (A1) telle que définie à l'une quelconque des revendications 1 à 18, et un autre une composition (B1) comprenant un agent oxydant dans un milieu approprié pour la teinture.

27. Dispositif à plusieurs compartiments ou 〈〈 Kit 〉〉 pour la teinture des fibres kératiniques et en particulier des fibres kératiniques humaines telles que les cheveux, caractérisé par le fait qu'il comporte au moins deux compartiments, dont l'un d'entre eux renferme une composition (A2) et un autre une composition (B2) telles que définies dans la revendication 25.

28. Utilisation d'une composition de teinture d'oxydation telle que définie à l'une quelconque des revendications 1 à 23 ou d'un dispositif de teinture ou 〈〈 Kit 〉〉 à plusieurs compartiments tel que défini à la revendication 26 ou 27, pour la teinture d'oxydation des fibres kératiniques humaines telles que les cheveux.

## Claims

1. Oxidation dye composition for keratin fibres, in particular for human keratin fibres such as the hair, comprising, in a medium which is suitable for dyeing, at least one oxidation dye precursor and, where appropriate, one or more couplers, characterized in that it also contains at least one anionic amphiphilic polymer containing at least one hydrophilic unit of formula (I) below: 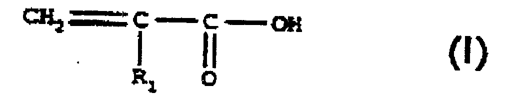 in which R₁ denotes H or CH₃ or C₂H₅, and at least one hydrophobic unit which is exclusively (C₁₀-C₃₀) alkyl ester of unsaturated carboxylic acid.

2. Composition according to Claim 1, characterized in that the hydrophilic unit of the anionic amphiphilic polymer is an acrylic acid, a methacrylic acid or mixtures thereof.

3. Composition according to Claim 1 or 2, characterized in that the hydrophobic unit of the anionic amphiphilic polymer is a (C₁₀-C₃₀) alkyl ester of unsaturated carboxylic acid of formula (II) below: 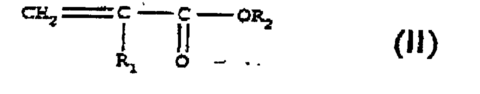 in which R₁ denotes H or CH₃ or C₂H₅, R₂ denoting a C₁₀-C₃₀ alkyl radical.

4. Composition according to Claim 3, characterized in that, in formula (II), R₁ denotes H or CH₃.

5. Composition according to Claims 3 or 4, characterized in that, in formula (II), R₂ denotes a C₁₂-C₂₂ alkyl radical.

6. Composition according to any one of the preceding claims, characterized in that the anionic amphiphilic polymer is crosslinked.

7. Composition according to any one of the preceding claims, characterized in that the anionic amphiphilic polymer is a polymer formed from a mixture of monomers essentially comprising acrylic acid, an ester of formula (II) below: 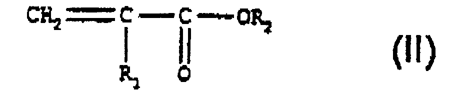 in which R₁ denotes H or CH₃, R₂ denoting an alkyl radical having from 12 to 22 carbon atoms, and a crosslinking agent.

8. Composition according to any one of Claims 1 to 5, characterized in that the anionic amphiphilic polymer is a polymer of acrylic acid and of lauryl methacrylate.

9. Composition according to any one of the preceding claims, characterized in that the oxidation dye precursors are chosen from ortho- or paraphenylenediamines, bis(phenyl)alkylenediamines, ortho- or para-aminophenols and heterocyclic bases, as well as the addition salts of these compounds with an acid.

10. Composition according to any one of the preceding claims, characterized in that the oxidation dye precursors are present in concentrations ranging from 0.0005 to 12% by weight relative to the total weight of the composition.

11. Composition according to any one of the preceding claims, characterized in that the couplers are chosen from meta-phenylenediamines, meta-aminophenols, meta-diphenols and heterocyclic couplers, and the addition salts of these compounds with an acid.

12. Composition according to any one of the preceding claims, characterized in that the couplers are present in concentrations ranging from 0.0001 to 10% by weight relative to the total weight of the composition.

13. Composition according to any one of Claims 9 to 12, characterized in that the addition salts with an acid of the oxidation dye precursors and of the couplers are chosen from the hydrochlorides, hydrobomides, sulphates, tartrates, lactates and acetates.

14. Composition according to any one of the preceding claims, characterized in that it also contains direct dyes.

15. Composition according to any one of the preceding claims, characterized in that it also contains at least one cationic or amphoteric substantive polymer.

16. Composition according to Claim 15, characterized in that the polymer is a quaternary polyammonium polymer consisting of repeating units corresponding to formula (VI) below:

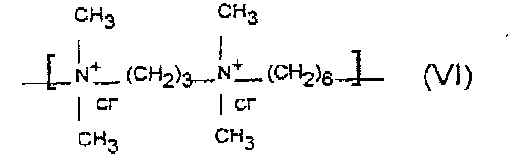

17. Composition according to Claim 15, characterized in that the polymer is a quaternary polyammonium polymer consisting of repeating units corresponding to formula (VII) below:

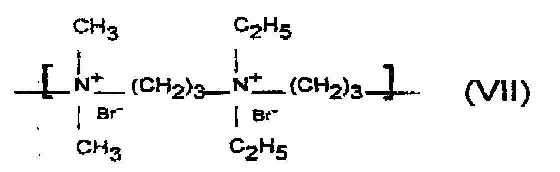

18. Composition according to any one of the preceding claims, characterized in that it also contains at least one antioxidant present in amounts ranging from 0.05 to 1.5% by weight relative to the total weight of the composition.

19. Ready-to-use composition according to any one of the preceding claims, characterized in that it also contains an oxidizing agent.

20. Composition according to Claim 19, characterized in that it has a pH ranging from 4 to 11.

21. Composition according to Claim 19 or 20, characterized in that the oxidizing agent is chosen from hydrogen peroxide, urea peroxide, alkali metal bromates and ferricyanides, and persalts.

22. Composition according to any one of Claims 19 or 21, characterized in that the oxidizing agent is an aqueous hydrogen peroxide solution whose titre ranges from 2.5 to 40 volumes.

23. Composition according to any one of the preceding claims, characterized in that the anionic amphiphilic polymers as defined in any one of Claims 1 to 8, are used in an amount ranging from 0.05 to 10% by weight relative to the total weight of the composition applied to the fibres, and even more preferably from 0.2 to 5%.

24. Process for dyeing keratin fibres, and in particular human keratin fibres such as the hair, characterized in that it consists in applying to the fibres a dye composition (A1) as defined in any one of Claims 1 to 18, and in developing the colour in alkaline, neutral or acidic medium using an oxidizing agent which is added, only at the time of use, to this composition (A1) or which is present in a composition (B1) that is applied sequentially without intermediate rinsing.

25. Process for dyeing keratin fibres, and in particular human keratin fibres such as the hair, characterized in that it consists in applying to the fibres a dye composition (A2) containing, in a medium which is suitable for dyeing, at least one oxidation dye precursor, optionally one or more couplers, in the presence or in the absence of an anionic amphiphilic polymer as defined in any one of Claims 1 to 8, and in developing the colour in alkaline, neutral or acidic medium using an oxidizing composition (B2) which contains an oxidizing agent and an effective amount of at least one anionic amphiphilic polymer as defined in any one of Claims 1 to 8, and which is mixed, only at the time of use, with the composition (A2) or which is applied sequentially without intermediate rinsing.

26. Multi-compartment device or "kit for dyeing keratin fibres, and in particular human keratin fibres such as the hair, characterized in that it contains at least two compartments, one of which contains a composition (A1) as defined in any one of Claims 1 to 18, and another of which contains a composition (B1) comprising an oxidizing agent in a medium which is suitable for dyeing.

27. Multi-compartment device or "kit" for dyeing keratin fibres, and in particular human keratin fibres such as the hair, characterized in that it contains at least two compartments, one of which contains a composition (A2) and another of which contains a composition (B2) as defined in Claim 25.

28. Use of an oxidation dye composition as defined in any one of Claims 1 to 23 or of a multi-compartment "kit" or device for dyeing as defined in Claim 26 or 27, for the oxidation dyeing of human keratin fibres

## Patentansprüche

1. Zusammensetzung zum oxidativen Färben von Keratinfasern und insbesondere menschlichen Keratinfasern, wie dem Haar, die in einem zum Färben geeigneten Medium mindestens ein Farbstoffvorprodukt eines Oxidationsfarbstoffes und gegebenenfalls einen oder mehrere Kuppler enthält, dadurch gekennzeichnet, daß sie ferner mindestes ein anionisches, amphiphiles Polymer enthält, das mindestens eine hydrophile Einheit der folgenden Formel (I) 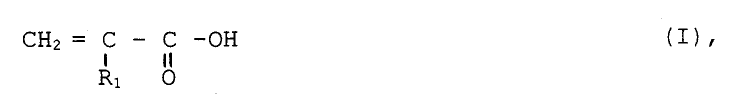 worin R₁ H, CH₃ oder C₂H₅ bedeutet,
und mindestens eine hydrophobe Einheit aufweist, bei der es sich ausschließlich um einen C₁₀₋₃₀-Alkylester einer ungesättigten Carbonsäure handelt.

2. Zusammensetzung nach Anspruch 1, dadurch gekennzeichnet, daß es sich bei der hydrophile Einheit des anionischen, amphiphilen Polymers um Acrylsäure, Methacrylsäure oder deren Gemische handelt.

3. Zusammensetzung nach Anspruch 1 oder 2, dadurch gekennzeichnet, daß die hydrophobe Einheit des anionischen, amphiphilen Polymers ein C₁₀₋₃₀-Alkylester einer ungesättigten Carbonsäure der folgenden Formel (II) ist: 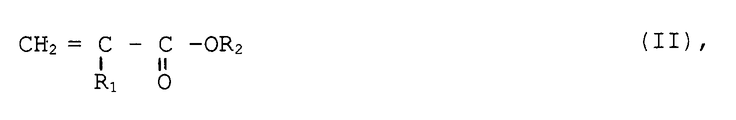 worin R₁ H oder CH₃ oder C₂H₅ und R₂ eine C₁₀₋₃₀-Alkylgruppe bedeutet.

4. Zusammensetzung nach Anspruch 3, dadurch gekennzeichnet, daß in Formel (II) R₁ H oder CH₃ bedeutet.

5. Zusammensetzung nach Anspruch 3 oder 5, dadurch gekennzeichnet, daß in Formel (II) R₂ eine C₁₂₋₂₂-Alkylgruppe bedeutet.

6. Zusammensetzung nach einem der vorhergehenden Ansprüche, dadurch gekennzeichnet, daß das anionische, amphiphile Polymer vernetzt ist.

7. Zusammensetzung nach einem der vorhergehenden Ansprüche, dadurch gekennzeichnet, daß das anionische, amphiphile Polymer ein Polymer ist, das aus einem Gemisch von Monomeren gebildet wird, das im wesentlichen Acrylsäure, einen Ester der folgenden Formel (II): 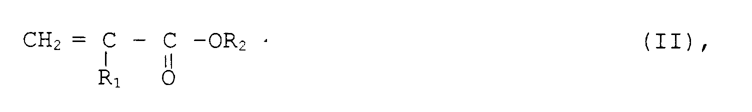 worin R₁ H oder CH₃ und R₂ eine Alkylgruppe mit 12 bis 22 Kohlenstoffatomen bedeutet, und ein Vernetzungsmittel enthält.

8. Zusammensetzung nach einem der Ansprüche 1 bis 5, dadurch gekennzeichnet, daß das anionische, amphiphile Polymer ein Polymer von Acrylsäure und Laurylmethacrylat ist.

9. Zusammensetzung nach einem der vorhergehenden Ansprüche, dadurch gekennzeichnet, daß die Farbstoffvorprodukte von Oxidationsfarbstoffen unter den o- oder p-Phenylendiaminen, Bisphenylalkylendiaminen, o- oder p-Aminophenolen, heterocyclischen Basen und den Additionssalzen dieser Verbindungen mit einer Säure ausgewählt sind.

10. Zusammensetzung nach einem der vorhergehenden Ansprüche, dadurch gekennzeichnet, daß die Farbstoffvorprodukte von Oxidationsfarbstoffen in Konzentrationen im Bereich von 0,0005 bis 12 Gew.-%, bezogen auf das Gesamtgewicht der Zusammensetzung, vorliegen.

11. Zusammensetzung nach einem der vorhergehenden Ansprüche, dadurch gekennzeichnet, daß die Kuppler unter den m-Phenylendiaminen, m-Aminophenolen, m-Dihydroxybenzolen, heterocyclischen Kupplern und den Additionssalzen dieser Verbindungen mit einer Säure ausgewählt sind.

12. Zusammensetzung nach einem der vorhergehenden Ansprüche, dadurch gekennzeichnet, daß die Kuppler in Konzentrationen im Bereich von 0,0001 bis 10 Gew.-%, bezogen auf das Gesamtgewicht der Zusammensetzung, vorliegen.

13. Zusammensetzung nach einem der Ansprüche 9 bis 12, dadurch gekennzeichnet, daß die Additionssalze der Farbstoffvorprodukte von Oxidationsfarbstoffen und der Kuppler mit einer Säure unter den Hydrochloriden, Hydrobromiden, Sulfaten, Tartraten, Lactaten und Acetaten ausgewählt sind.

14. Zusammensetzung nach einem der vorhergehenden Ansprüche, dadurch gekennzeichnet, daß sie ferner Direktfarbstoffe enthält.

15. Zusammensetzung nach einem der vorhergehenden Ansprüche, dadurch gekennzeichnet, daß sie ferner mindestens ein kationisches oder amphoteres substantives Polymer enthält.

16. Zusammensetzung nach Anspruch 15, dadurch gekennzeichnet, daß das Polymer ein quartäres Polyammoniumpolymer ist, das aus wiederkehrenden Einheiten der folgenden Formel (VI) besteht:

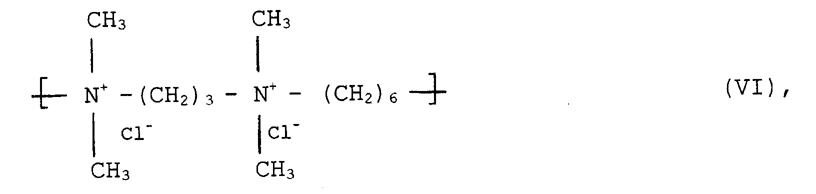

17. Zusammensetzung nach Anspruch 15, dadurch gekennzeichnet, daß das Polymer ein quartäres Polyammoniumpolymer ist, das aus wiederkehrenden Einheiten der folgenden Formel (VII) besteht:

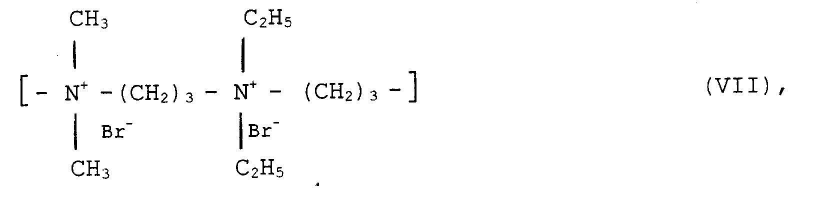

18. Zusammensetzung nach einem der vorhergehenden Ansprüche, dadurch gekennzeichnet, daß sie ferner mindestens ein Oxidationsmittel enthält, das in Mengenanteilen von 0,05 bis 1,5 Gew.-%, bezogen auf das Gesamtgewicht der Zusammensetzung vorliegt.

19. Zusammensetzung nach einem der vorhergehenden Ansprüche, die gebrauchsfertig ist, dadurch gekennzeichnet, daß sie ferner mindestens ein Oxidationsmittel enthält.

20. Zusammensetzung nach Anspruch 19, dadurch gekennzeichnet, daß sie einen pH-Wert im Bereich von 4 bis 11 aufweist.

21. Zusammensetzung nach Anspruch 19 oder 20, dadurch gekennzeichnet, das das Oxidationsmittel unter Wasserstoffperoxid, Harnstoffperoxid, Bromaten oder Ferricyaniden von Alkalimetallen und Salzen von Persäuren ausgewählt ist.

22. Zusammensetzung nach einem der Ansprüche 19 bis 21, dadurch gekennzeichnet, daß das Oxidationsmittel eine Wasserstoffperoxidlösung mit einem Titer im Bereich von 2,5 bis 40 Volumina ist.

23. Zusammensetzung nach einem der vorhergehenden Ansprüche, dadurch gekennzeichnet, das die anionischen, amphiphilen Polymere nach einem der Ansprüche 1 bis 8 in einem Mengenanteil im Bereich von 0,05 bis 10 Gew.-% des Gesamtgewichts der auf die Fasern aufgebrachten Zusammensetzung und noch bevorzugter im Bereich von 0,2 bis 5 Gew.-% verwendet werden.

24. Verfahren zum Färben von Keratinfasern und insbesondere menschlichen Keratinfasern, wie dem Haar, dadurch gekennzeichnet, daß es darin besteht, auf die Fasern mindestens eine Zusammensetzung (A1) nach einem der Ansprüche 1 bis 18 aufzutragen und die Farbe bei einem alkalischen, neutralen oder sauren pH-Wert mit einem Oxidationsmittel zu entwickeln, das bei der Anwendung mit der Zusammensetzung (A1) vermischt wird oder das in einer Zusammensetzung (B1) vorliegt, die getrennt davon anschließend ohne zwischenzeitliches Spülen aufgetragen wird.

25. Verfahren zum Färben von Keratinfasern und insbesondere menschlichen Keratinfasern, wie dem Haar, dadurch gekennzeichnet, daß es darin besteht, auf die Fasern eine Zusammensetzung (A2) aufzutragen, die in einem zum Färben geeigneten Medium mindestens ein Farbstoffvorprodukt eines Oxidationsfarbstoffes und gegebenenfalls einen oder mehrere Kuppler in Gegenwart oder Abwesenheit eines anionischen, amphiphilen Polymers nach einem der Ansprüche 1 bis 8 enthält, und die Farbe bei einem alkalischen, neutralen oder sauren pH-Wert mit einer oxidierenden Zusammensetzung (B2) zu entwickeln, die ein Oxidationsmittel und eine wirksame Menge mindestens eines anionischen, amphiphilen Polymers nach einem der Ansprüche 1 bis 8 enthält und die bei der Anwendung mit der Zusammensetzung (A2) vermischt wird oder getrennt davon anschließend ohne zwischenzeitliches Spülen aufgetragen wird.

26. Vorrichtung mit mehreren Abteilungen oder 〈〈Kit〉〉 zum Färben von Keratinfasern und insbesondere menschlichen Keratinfasern, wie dem Haar, dadurch gekennzeichnet, daß sie mindestens 2 Abteilungen aufweist, wobei eine Abteilung eine Zusammensetzung (A1) nach einem der Ansprüche 1 bis 18 und eine weitere Abteilung eine Zusammensetzung (B1) enthält, die in einem zum Färben geeigneten Medium das Oxidationsmittel enthält.

27. Vorrichtung mit mehreren Abteilungen oder 〈〈Kit〉〉 zum Färben von Keratinfasern und insbesondere menschlichen Keratinfasern, wie dem Haar, dadurch gekennzeichnet, daß sie mindestens 2 Abteilungen aufweist, wobei eine Abteilung eine Zusammensetzung (A2) und eine weitere Abteilung eine Zusammensetzung (B2) nach Anspruch 25 enthält.

28. Verwendung einer Zusammensetzung zum oxidativen Färben nach einem der Ansprüche 1 bis 23 oder einer Vorrichtung oder eines 〈〈Kits〉〉 mit mehreren Abteilungen nach einem der Ansprüche 26 oder 27 zum oxidativen Färben von menschlichen Keratinfasern, wie dem Haar.
